Europäisches Patentamt

European Patent Office

Office européen des brevets

(19)

(11) Publication number: **0 192 440**
A2

# (12) EUROPEAN PATENT APPLICATION

(21) Application number: 86301055.9

(22) Date of filing: 17.02.86

(51) Int. Cl.⁴: **A 61 B 17/60**

(30) Priority: 21.02.85 GB 8504539

(43) Date of publication of application:
27.08.86 Bulletin 86/35

(84) Designated Contracting States:
AT BE CH DE FR GB IT LI LU NL SE

(71) Applicant: Chas F Thackray Limited
P.O. Box HP 171 1 Shire Oak Street
Leeds LS6 2DP West Yorkshire(GB)

(72) Inventor: Shelley, Philip
35 Runshaw Avenue
Appleybridge Wigan, WN6 9JP(GB)

(72) Inventor: Shearer, John Robertson
Quince Cottate Dean
Bishops Waltham Southampton, S03 1FY(GB)

(74) Representative: Harrison, Michael Robert et al,
Urquhart-Dykes & Lord 5th Floor Tower House Merrion
Way
Leeds, LS2 8PA(GB)

(54) Improved bone fixation system.

(57) An external bone fixation system comprises two tubular
members (38, 39) joined by an articulation block (40, 37).
Each pin carrier holds a bone fixation pin (17) for inserting
into the bone, and the pin carriers are adapted so that two
adjacent pins may be inserted into the bone with a minimum
axial separation of no more than 1cm, and with a maximum
included angle (B) of at least 30°.

FIG. 7.

# IMPROVED BONE FIXATION SYSTEM

This invention relates to a system for the immobilisation of one bone fragment relative to another bone fragment, which may be used for treatment of bone fractures, for leg lengthening, or for arthrodesis of joints, ie. the immobilisation of joints by removal of the cartilaginous surfaces so that the bones grow solidly together. Such a system hereinafter will be referred to as "an external bone fixation system".

A known simple form of external fixation system is a single rod onto which is cemented or clamped the ends of pins, the other ends of these pins being inserted into the bone fragments. This type of device does immobilise the bone fragments but does not allow misaligned bone fragments to be brought into the correct alignment by translational, rotational, or angular movement, or a combination of any of these after immobilisation.

There are some currently known devices which do allow some sort of relative movement between adjacent bone fragments to correct misalignment, but such gains in versitility are usually accompanied by increases in costs and bulk.

Furthermore, currently available single sided external bone fixation systems are such that all the bone fixation pins must be inserted into the bone either in the same plane, or with a small angle between the planes defined by the longitudinal axes of alternate pins. This angle is too small to give adequate protection against torsional and bending forces, and also suffers from the disadvantage that all the pins may work loose as a result of any forces tending to pull the pins away from the bone.

French Patent Application No 7914508 (Publication No 2457676) described a single sided external bone fixation system which is intended to replace a temporary external support. The temporary support allows a number of groups of pins to be placed in a fractured bone, one group of pins in each bone fragment, all of the pins being held by the support in the same place. The fixation system retains the positions of the pins in the bone, and comprises a tube which carries two slidable collars. Each of the collars accommodates a separate tubular portion for accommodating the protruding ends of a group of pins. A ball and socket joint between each tubular portion carrying the pins and its associated collar allows the previously immobilised bone fragments to be brought into correct alignment so that the fracture can heal. This system does not allow any flexibility of pin position or angle of insertion, since these are determined by the temporary support which only allows the pins to be inserted into the bone at right angles to the axis of the support, all the pins being in the same plane.

It is the purpose of the present invention to provide a low cost, simple and effective external fixation system which allows correction of bone misalignment and provides improved protection against torsional and bending forces.

According to the present invention there is provided an external bone fixation system including at least one elongate member for coupling to a single bone fragment, at least two pin carriers mountable on the elongate member and adjustable thereon between a first condition in which the pin carrier may rotate about and slide along the elongate member and a second condition in which the pin carrier 15 is rigidly fixed to the elongate member, each pin carrier having means at one end thereof rigidly to hold a bone fixation pin and being adapted so as to allow at least two adjacent pins to be inserted into the single bone fragment with a minimum axial separation of not greater than 1cm and with a maximum included angle between the longitudinal axes of said adjacent pins of at least 30°.

By a "single bone fragment" is meant a piece of bone which has been separated from the rest of that bone, or equally a whole bone such as a femur or tibia.

In practice, with a fixation system according to the present invention, the included angle between the longitudinal axes of adjacent pins may be anything from $0^{\circ}$ to $90^{\circ}$, the latter being achieved by the use of a modified pin carrier.

Preferably the included angle is at least $40^{\circ}$, although it should be appreciated that the angle is in fact dependent to a certain extent on the type and size of bone, and on the length of pins used.

More preferably, the included angle is $50^{\circ}$, the most preferred included angle being $60^{\circ}$.

The minimum axial separation when two standard pin carriers are used adjacent each other is 0.7cm, but this can be reduced to zero if a modified pin carrier is used in place of one of the standard carriers.

An advantage of the present invention is that any number of pins may thus be inserted into the bone fragment, depending on the size of the fragment.

Preferably the bone fixation system also comprises an articulation block for mounting between a first elongate member coupled to a first bone fragment and a second elongate member coupled to a second bone fragment, the articulation block having at least one bore for accommodating an end of the first elongate member and at least one other bore for accommodating an end of the second elongate member, at least one of said elongate members having attached at said end means to allow the elongate members to be angularly and rotationally moved with respect to each other.

Preferably the means for allowing the elongate members to be angularly and rotationally moved with respect to each other is a ball for locating within the articulation block.

Thus the articulation block allows the two elongate members to be moved allowing both parallax and angular correction of the elongate members to allow angular or parallax correction of the bones or bone fragments in addition to distraction or compression. This is the only single-joint system which allows such flexibility.

The system is a modular system and can therefore be used to deal with multiple fractures, each discrete fracture having a corresponding articulation block.

Preferably each pin carrier comprises a first block including at one end a semi-cylindrical cut-out, a second block including a matching semi-cylindrical cut-out, the cylindrical bore so produced being capable of slidably and rotatably accommodating a first or second elongate member in accordance with the present invention, the first and second blocks each including at least two aligned bores for accommodating tightening screws, the pin carrier also including end members having aligned bores for placing at each end of the pin carrier, the end of the pin carrier being adapted so as to accommodate a bone fixation pin such that an end member may be tightened against the end of the pin carrier to hold the pin in position in the pin carrier.

Thus the pin is held in place in the pin carrier which can then be moved up or down or rotated with respect to the or each elongate members before being tightened in position. Each elongate member can carry numerous pin carriers so that a complex bone fracture can be dealt with. The pin carrier itself acts as a drilling guide and so removes the need for the use of drilling jigs.

There may also be provided an improved bone fixation pin in which the point of the tapered end is planar with a self-drilling cutting edge and clearance angle so that the pin is easier to insert and at the same time causes minimal thermal damage to the bone. The point is also designed to reduce the risk of neurological damage during insertion of the pin. The pin has a sharp and thin thread form with a long thread pitch. This reduces pin insertion torque but more importantly maximises bone thread volume and so pin pull-out strength.

Since each pin carrier can be rotated with respect to the or each elongate member pin position is variable so as to avoid areas of doubtful skin viability or to leave access for vascular or plastic surgeons.

One of the major problems with any type of fixation device for healing a bone fracture is that there is a reduction in load transmission within the treated bone. Since it is known that bone regrowth is stronger when the bone is loaded, some devices have been developed which artificially exert force on the bone, however these systems are often complex.

According to the present invention there may also be provided a first easily detachable device for allowing, when fitted to the fixation system the two elongate members to rotate and slide axially with respect to each other, so that torsional forces and axial loading may be transmitted through the bone.

Preferably, there is also provided a second easily detachable device for allowing, when fitted to the fixation system in addition to the first device, the two elongate members to slide axially with respect to each other whilst preventing relative rotation therebetween, so that axial loading only is transmitted through the bone.

According to the present invention there may also be provided a wrench having means for loosening and tightening all the screws within the bone fixation system of the present invention.

Embodiments of the present invention will now be described, by way of example only, with reference to the accompanying drawings in which:

Figure 1 is an exploded diagram of an articulation block in accordance with the present invention; **0192440**

Figure 2 is an exploded diagram of a pin carrier in accordance with the present invention;

Figure 3 is an angled pin carrier in accordance with the present invention;

Figure 3a shows the grooves in the pin carrier shown in Figure 3 for accommodating a bone fixation pin;

Figure 4 is an angled pivoted pin carrier in accordance with the present invention;

Figure 5 is an improved bone fixation pin in accordance with the present invention;

Figure 5a is a close-up of the thread pattern of the bone fixation pin shown in Figure 6;

Figure 5b is a close-up of the tapered end of the bone fixation pin shown in Figure 6;

Figure 6 is a plan view of an antirotation device;

Figure 6b is a perspective view of the device shown in Figure 6;

Figure 7 shows the use of the present invention for fixing a simple fracture;

Figure 8 is a side view of the fixation system shown in Figure 7;

Figure 9 shows the use of the present invention for fixing a butterfly fracture, using an angled pin carrier;

Figure 10 shows an alternative arrangement of the present invention for use in treating pelvic fractures or for limb immobilisation; and,

Figure 11 is a wrench for assembling and dissembling the bone fixation system of the present invention.

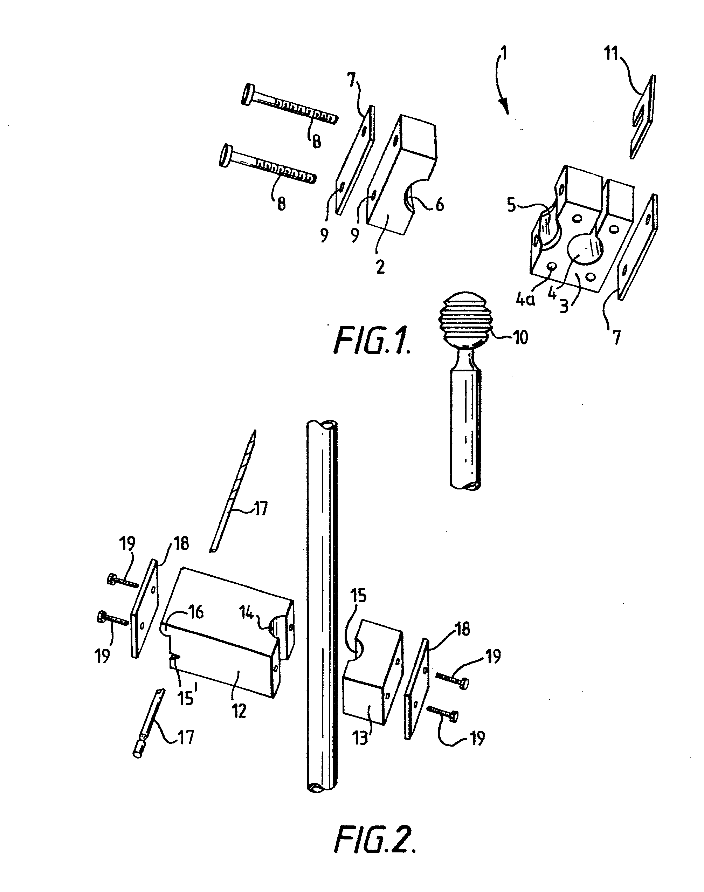

With reference to Figure 1 a standard articulation block 1 comprises two sections 2 and 3. Section 3 has a split structure with a central bore 4 for accommodating a first tube. Section 3 also has at one end a recess 5. Section 2 also includes at one end a recess 6. End plates 7 and locking screws 8 are provided such that the articulation block 1 can be assembled with the two recesses 5 and 6 in sections 3 and 2 respectively lying one on either side of a grooved articulation ball either split or solid at one end of a second tube. The end of the ball may be free or attached to the second tube by brazing or welding. This standard articulation block permits two tubes to be joined in-line whilst permitting a degree of angulation, typically 25 degrees, in any plane. Rotation of both tubes is unlimited until the joint is fully tightened. Preferred materials are dense plastic for sections 2 and 3, metal for the end plates 7 and steel for the locking screws 8.

A spacer device 11 may be inserted in the slot of section 3 to prevent tightening of section 3 around the first tube, thus allowing rotation and sliding of the first tube with respect to the second.

An alternative type of articulation block, for use as shown in Figure 10 permits two tubes to be clamped at 90 degrees to each other, with typically 25 degrees articulation of one tube with respect to the other still being possible in any plane. Using this type of articulation block it is possible to treat a pelvic fracture or immobilise two limbs with respect to each other for skin grafting by having one horizontal tube 48 having articulation ball 49 at one of its ends locating within an alternative articulation block 50. Each articulation block 50 clamps a second tube 51 at 90 degrees to the first tube 48, the second tube 51 having a number of pin carriers 52 mounted along its length. Alternatively, tube 48 may be provided without an articulation ball in which case both tubes 51 would have articulation balls which locate in blocks 50.

With reference to Figure 2 a standard pin carrier comprises two blocks 12 and 13, typically of dense plastic, with recesses 14 and 15 cut into oppositely facing ends.

At the end of block 12 remotest from recess 14 there is a longitudinal groove 15 for accommodating a bone fixation pin 17. Metal end plates 18 are secured one at each end of blocks 12 and 13 by steel locking screws 19, such that a longitudinal overhang 16 on block 12 forms an abutment for the face of end plate 18. When the locking screws are fully tightened the blocks 12 and 13 are clamped with recesses 14 and 15 one on either side of a tube, so that no rotation or sliding of the pin carrier with respect to the tube is possible. The groove 15 is placed to one side of the block 12 such that two pins on two pin carriers can be positioned within a small axial distance, typically 0.7cm of each other by positioning the blocks accordingly. This feature is particularly useful for fractures occuring at the end of bones, where space is limited.

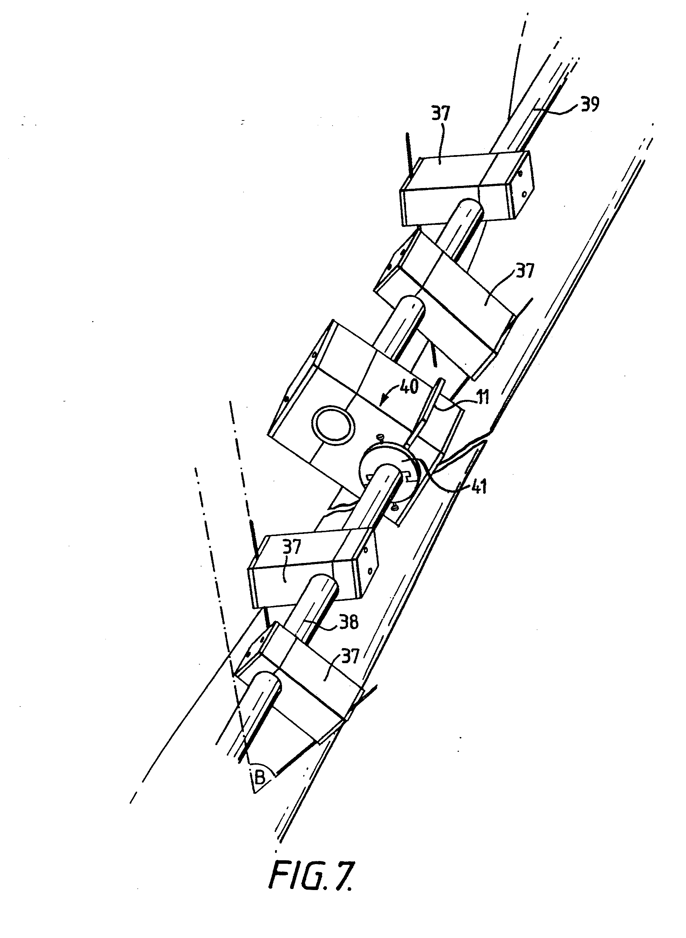

The design of the pin carrier is such that the distance between the bone fixation pin 17 and the tube on which the pin carrier is mounted allows the angle between the two planes defined by the longitudinal axes of alternate fixation pins (this angle is marked B in Figure 7) to be as near to 90 degrees as possible in order to maximise bending and torsional rigidity of the fixation system. In practise any angle of between 0 degrees and 90 degrees can be achieved, depending upon the length of the fixation pins and the soft tissue thickness over the bones. An angle of at least 30 degrees is usually achievable whereas a larger angle such as 40 degrees is preferable. A more preferable angle is $50^{\circ}$, and $60^{\circ}$ is the most prefered angle. The maximum angle between the axes of adjacent fixation pins should be at least 30 degrees for 180mm long pins and 35 degrees for 150mm long pins. This arrangement also has the advantage that it would not be possible for all of the fixation pins to work loose at the same time, thus enhancing the stability of the system, should forces be applied tending to pull the pins out of the bone. These

0192440

minimum angles are for the standard size bone fixation system, but it should be appreciated that the whole bone fixation system may be scaled down in size with corresponding minimum angles for the smaller fixation system and the shorter pins.

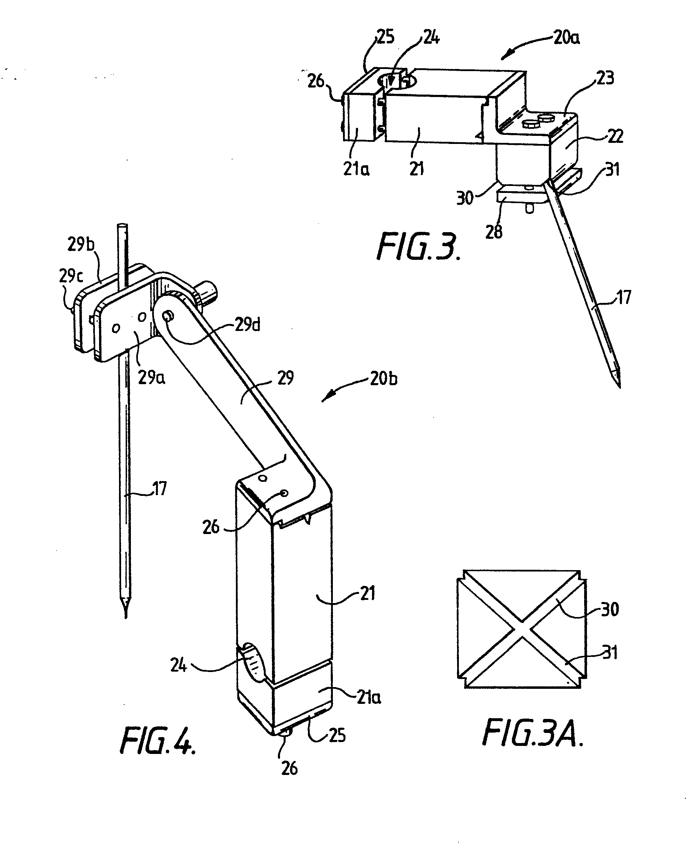

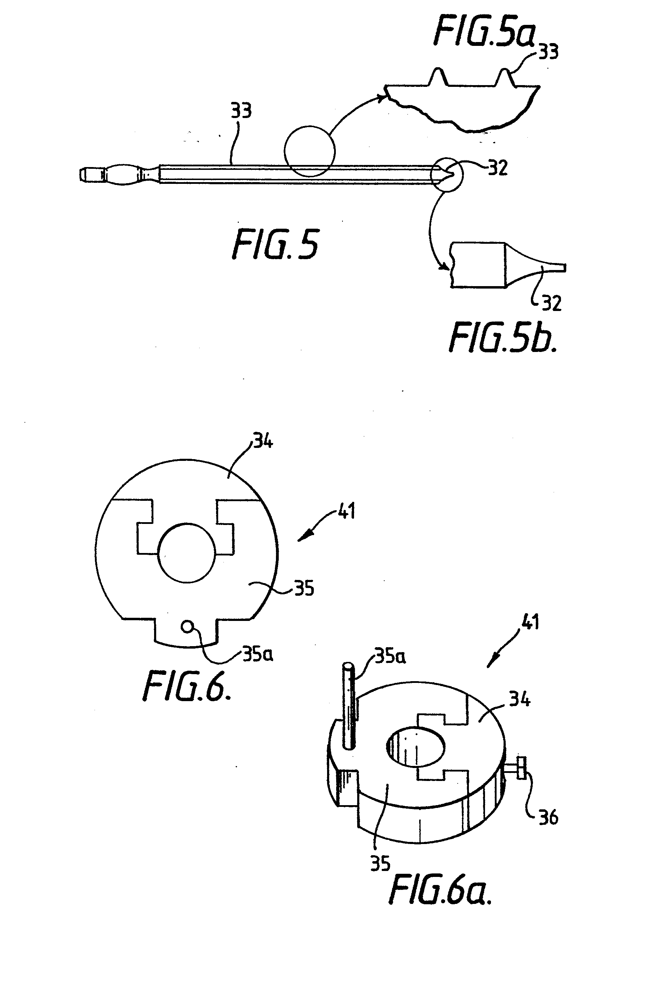

With reference to Figure 3 a modified pin carrier 20a permits insertion of a fixation pin in the same axial position as an existing pin carrier or articulation block. This pin carrier comprises three blocks 21, 21a and 22 all made of dense plastic. Blocks 21 and 21a form a cylindrical bore 24 to receive a tube. A metal end plate 25 has aligned tightening bores which allow the blocks 21 and 21a to be clamped to a tube via locking screws 26. Block 22 has at one end two diagonally running pin grooves 30, 31 as shown in Figure 5 such that a fixation pin may be secured into either groove, thus giving greater choice of pin position, by tightening metal end plate 28 against the block 22. Blocks 21 and 22 are fixed at 90 degrees with respect to each other by a metal bracket 23 secured by locking screws at blocks 21 and 22. Blocks 21 and 21a and end plate 25 and screws 26 are all as for a standard pin carrier as shown in Figure 2.

With reference to Figure 4 a further alternative pin carrier 20b permits a fixation pin to be inserted at a larger included angle than usual, for example 90°. This facility is useful when there is soft tissue damage which dictates fixation pin position or when the pin has to be resited following a pin-track infection. The pin carrier comprises two blocks 21 and 21a of dense plastic and two metal brackets 29 and 29a. Blocks 21, 21a and end plate 25 and screws 26 are all as for a standard pin carrier as shown in Figure 2. Angled bracket 29 is clamped to block 21 by locking screws 26. Bracket 29a is mounted pivotally on bracket 29 and has at one end a metal end plate 29b and locking screws 29c which can be tightened to clamp a fixation pin between end plate 29b and the end plate of bracket 29a. The angle of the fixation pin between end plate 29b and the end plate of bracket 29a is not fixed by a groove, and can be chosen to suit the prevailing soft tissue conditions.

With reference to Figures 5, 5a and 5b an improved bone fixation pin is provided having a blunt drilling head 32 and a widely spaced thread pattern 33. The head 32 has a self-drilling cutting edge and clearance angle which makes the pin easier to insert and minimise thermal damage to the bone. The design of the pin also reduces the risk of neurological damage resulting from pin insertion. The long pitch thread pattern 33 results in improved contact between the pin surface and the bone and hence improved pin pull-out strength. The diameter of the pin is typically 4mm-6.5mm for a large fixator, and 2mm-4mm for a small fixator.

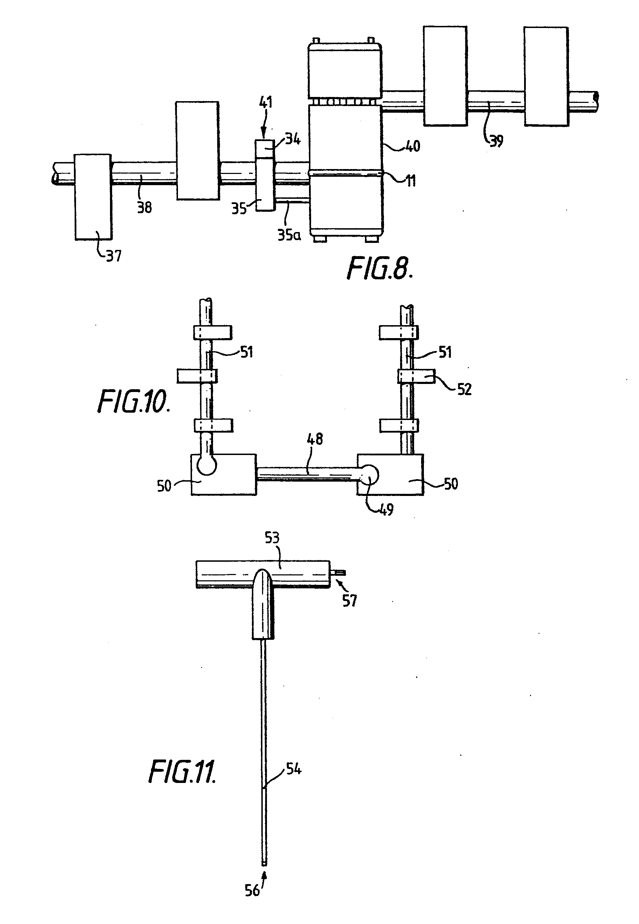

Referring to Figures 1 to 7, axial load may be transmitted through the fractured bone by inserting a spacer 11 into the articulation block to allow it to slide up and down the tube. However, the insertion of the spacer means that the articulation block is also free to rotate about the tube. In the early stages of healing of the fracture, it is desirable to allow limited axial loading whilst preventing any torsional forces which might impair the healing of the fracture. This is achieved by affixing to the tube, adjacent to the articulation block an antirotation device 41 such as that shown in Figures 7 and 8. This comprises two sections 34 and 35 for clamping using locking screws 36 about the tube adjacent the articulation block 40. Section 35 includes a peg 35a for insertion within one of the holes 4a of the articulation block (figure 1). When the device is fitted, along with the spacer 11, an increased proportion of the axial physiological load is transmitted through the fracture whilst avoiding damaging torsional forces.

11

The antirotation device 41 also serves as a stop to limit the relative axial movement of the two tubes, the position of device on the tube determining the possible amount of such movement.

If required, torsional load, may also be transmitted through the fracture by the removal of the antirotation device 41 whilst retaining the spacer 11 in place within the articulation block, so that the articulation block is free to rotate about and slide along the tube. These devices 41 and 11 give facilities for varying the stability of the fixation system according to bone healing requirements.

With reference to Figures 7 and 8 numerous pin carriers 37 positioned at intervals along tubes 38 and 39, these tubes being connected by a standard articulation block 40, are used to fix a simple or primary fracture in a single sided arrangement. The antirotation device 41 and spacer 11 are shown positioned adjacent to the articulation block 40 in order to allow axial loading of the fracture. If the surgeon chooses, a double sided fixation arrangement may be used, with a similar arrangement such as that shown in Figure 8 also on the opposite side of the bone. The antirotation device, when fitted, also serves to limit the range of axial movement of the two tubes 38, 39 as described above.

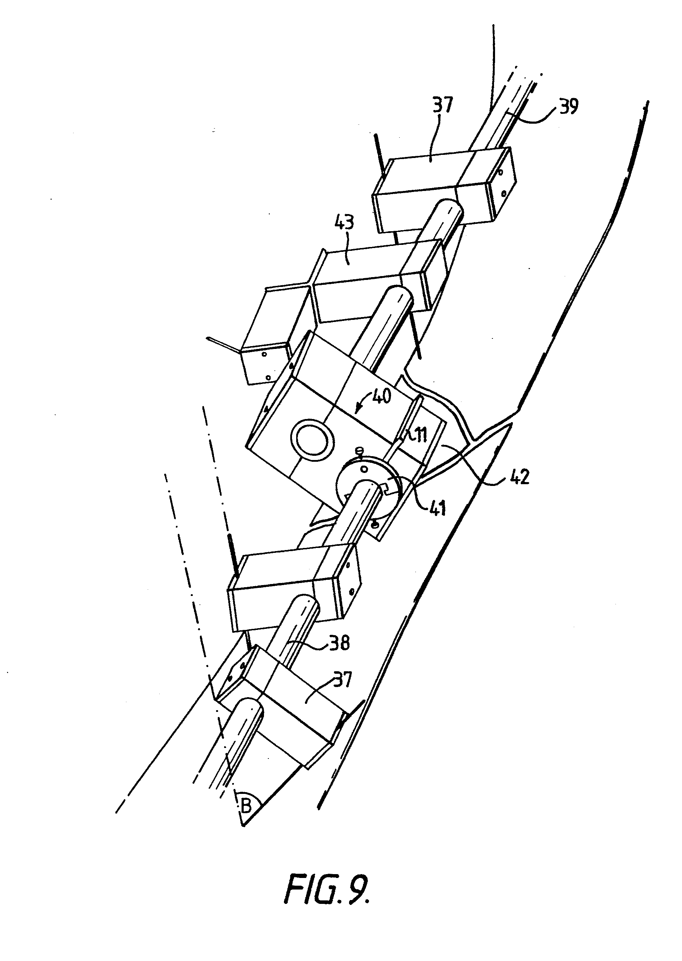

With reference to Figure 9 fixation of a butterfly fracture is shown using several standard pin carriers 37 mounted on tubes 38 and 39, the tubes 38 and 39 being linked by a standard articulation block 40 which happens to lie above a separate fragment of bone 42 to be fixed. An angled pin carrier 43 as shown in Figure 3 allows a fixation pin to be inserted into the separate fragment of bone at the same axial position as the articulation block, and so the fragment may be fixed between the two larger segments.

0192440

It should be appreciated that in the present invention any number of fixation pins can be placed, limited only by the number of pin carriers that may be placed upon the structure.

Also it should be appreciated that the present invention may be enhanced by adding additional articulation blocks to cater for complex segmented or pelvic fractures as shown in Figure 10. When so used, the present invention allows individual testing of each fracture for bone union, in isolation from all other fractures.

It is important if electrical stimulation of bone union is employed that the pins are electrically insulated both from each other and the tubes, and this is achieved by the use of plastic for the pin carriers and articulation block.

The fixator may also be used single-sided or double-sided without an articulation block. In this instance a single tube would be used with pin carriers clamped along its length.

With reference to Figure 11, a wrench for tightening and loosening all the screws within the bone fixation system has a handle 53, a shaft 54 which has at the end remote from the handle an allen key 56 of a size suited to the heads of the screws in the system is brazed. This wrench may also take the form of a box-spanner in the event that hexagonal-headed screws are to be used. The allen key end 57 of the handle 53 may be used to tighten and loosen any screws not easily accessed by the allen key 56.

CLAIMS

1. An external bone fixation system including at least one elongate member (38) for coupling to a bone fragment characterised in that it includes, at least two pin carriers (37) mountable on the elongate member and adjustable thereon between a first condition in which the pin carrier may rotate about and slide along the elongate member and a second condition in which the pin carrier is rigidly fixed to the elongate member, each pin carrier having means (15, 30, 31) at one end thereof rigidly to hold a bone fixation pin and being adapted so as to allow at least two adjacent pins to be inserted into the single bone fragment with a minimum axial separation of not greater than 1cm and with a maximum included angle (B) between the longitudinal axes of said adjacent pins of at least 30°.

2. An external bone fixation system according to Claim 1 characterised in that it includes an articulation block (1) for mounting between a first elongate member (38) coupled to a first bone fragment and a second elongate member (39) coupled to a second bone fragment, the articulation block having at least one bore (4) for accommodating an end of the first elongate member (38) and at least one other bore (5, 6) for accommodating an end of the second elongate member (39), at least one of said elongate members having attached at said end means (10) to allow the elongate members to be angularly and rotationally moved with respect to each other.

3. An external bone fixation system according to Claim 1 or Claim 2 characterised in that each pin carrier comprises a first block (13) including at one end a semi-cylindrical cut-out (15), a second block (12) including a matching semi-cylindrical cut-out (14), the cylindrical bore so produced being capable of slidably and rotatably accommodating said first or second elongate member, the first and second blocks each including at least two aligned bores for accommodating tightening screws (19), the pin carrier also including end members (18) having aligned bores for placing at each end of the pin carrier, the end of the pin carrier being adapted so as to accommodate a bone fixation pin such that an end member may be tightened against the end of the pin carrier to hold the pin in position in the pin carrier.

4. An external bone fixation system according to any of the preceding claims characterised in that the bone fixation pin (17) has a tapered end (32) of which the point is planar with a self-drilling cutting edge and clearance angle.

5. An external bone fixation system according to any of Claims 2 to 4 characterised in that it includes a first easily detachable device (11) for allowing, when fitted to the fixation system, the two elongate members to rotate and slide axially with respect to each other, so that both torsional forces and axial loading may be transmitted through the bone.

6. An external bone fixation system according to Claim 5, characterised in that it includes a second easily detachable device (41) for allowing, when fitted to the fixation system in addition to the first device (11), the two elongate members to slide axially with respect to each other whilst preventing relative rotation therebetween, so that axial loading only is transmitted through the bone.

7. An external bone fixation system according to any of the preceding claims characterised in that it includes a wrench having means (56, 57) for loosening and tightening all the screws within the bone fixation system.

8. An external bone fixation system according to any of the preceding claims characterised in that it includes a first modified pin carrier (20b) for allowing two adjacent

0192440

pins to be inserted into the bone fragment with an included angle between the respective longitudinal axes of the pins of up to 90°.

9. An external bone fixation system according to any of the preceding claims characterised in that it includes a second modified pin carrier (20a) for allowing two adjacent pins to be inserted into the bone fragment with an axial separation of 0cm or more.

FIG.1.

FIG.2.

FIG.3.

FIG.4.

FIG.3A.

FIG.5

FIG.5a

FIG.5b.

FIG.6.

FIG.6a.

FIG. 7.

0192440

FIG.8.

FIG.10.

FIG.11.

FIG.9.